# EUROPEAN PATENT SPECIFICATION

(11) **EP 3 761 904 B1**
(45) Date of publication and mention of the grant of the patent: **28.02.2024**
(21) Application number: 19723855.3
(22) Date of filing: 05.04.2019
(51) Int. Cl.: A61F 2/16

(54) **HYBRID OPTICAL EDGE FOR AN INTRAOCULAR LENS (IOL)**
HYBRIDE OPTISCHE KANTE FÜR EINE INTRAOKULARLINSE (IOL)
BORD OPTIQUE HYBRIDE POUR LENTILLE INTRAOCULAIRE (IOL)

(30) Priority: 06.04.2018 US 201862653588 P
(43) Date of publication of application: 13.01.2021
(73) Proprietor: Alcon Inc., 1701 Fribourg (CH)
(72) Inventor: DAS, Kamal D., Fort Worth, Texas 76134 (US); LIU, Jian, Keller, Texas 76248 (US)
(74) Representative: Murgitroyd & Company
(86) International application number: PCT/IB2019/052826
(87) International publication number: WO 2019/193569

(56) References cited:
- EP-A1- 2 030 594
- EP-B1- 2 030 594
- WO-A1-2008/036674
- WO-A1-2014/088716
- WO-A2-2005/055875
- US-A1- 2004 059 414

## Description

### FIELD

The present disclosure relates to intraocular lenses (IOLs), and more specifically, to a hybrid optical edge for an IOL.

### BACKGROUND

The human eye includes a cornea and a crystalline lens that are intended to focus light that enters the pupil of the eye onto the retina. However, the eye may exhibit various refractive errors which result in light not being properly focused upon the retina, and which may reduce visual acuity. Ocular aberrations can range from the relatively simple spherical and cylindrical errors that cause myopia, hyperopia, or regular astigmatism, to more complex refractive errors that can cause, for example, halos and starbursts in a person's vision.

Many interventions have been developed over the years to correct various ocular aberrations. These include spectacles, contact lenses, corneal refractive surgery, such as laser-assisted in situ keratomileusis (LASIK) or corneal implants, and intraocular lenses (IOLs). The diagnosis and specification of sphero-cylindrical spectacles and contact lenses for treatment of myopia, hyperopia, and astigmatism are also well-established.

In particular, it has been observed that edge portions of implanted IOLs may result in complex refractive errors that can cause undesired effects, such as glare and other undesired refraction of incoming light, which may adversely affect the quality of vision using the implanted IOL.

Reference is made to EP2030594A1 which has been cited in the search report as relating to the state of the art. This document teaches lens arrangements including a peripheral edge circumscribing the optic faces and provision of regions separating the edge from the optical surfaces.

### SUMMARY

The invention is defined by the claims.

In certain embodiments of the present disclosure, an intraocular lens includes an optic having an anterior optic surface, a posterior optic surface, and an optic edge extending between the anterior optic surface and a posterior optic surface. The optic edge includes a convex portion extending from a periphery of the anterior optic surface and a flat portion extending from a periphery of the posterior optic surface such that a corner edge is formed between the optic edge and the posterior optic surface. The flat portion intersecting the convex portion to form the optic edge. Such an optic edge may eliminate glare or other undesired effects that adversely impact quality of vision.

### BRIEF DESCRIPTION OF THE DRAWINGS

For a more complete understanding of the present invention and its features and advantages, reference is now made to the following description, taken in conjunction with the accompanying drawings, in which:
FIGURE 1 is a depiction of an IOL;
FIGURE 2 is a depiction of IOL edges;
FIGURE 3 is a depiction of IOL edge effects;
FIGURE 4 is a data plot showing IOL edge transmission versus azimuthal angle of incidence;
FIGURE 5 is a depiction of an IOL having a hybrid edge design;
FIGURE 6 is a depiction of the effects of an IOL having a hybrid edge design; and
FIGURE 7 is a data plot showing edge transmission versus azimuthal angle of incidence for an IOL having a hybrid edge design.

### DETAILED DESCRIPTION

In the following description, details are set forth by way of example to facilitate discussion of the disclosed subject matter. It should be apparent to a person of ordinary skill in the field, however, that the disclosed implementations are exemplary and not exhaustive of all possible implementations.

Throughout this disclosure, a hyphenated form of a reference numeral refers to a specific instance of an element and the un-hyphenated form of the reference numeral refers to the element generically or collectively. Thus, as an example (not shown in the drawings), device "12-1" refers to an instance of a device class, which may be referred to collectively as devices "12" and any one of which may be referred to generically as a device "12". In the figures and the description, like numerals are intended to represent like elements.

As noted, edge portions of implanted IOLs have been observed to result in complex refractive errors that can cause undesired effects, such as glare and other undesired refraction of incoming light, which may adversely affect the quality of vision using the implanted IOL. Glare (also known as positive dysphotopsia) may be associated with brightness or streaks of light, which is considered as unwanted light or a visual disturbance for an implantee of an IOL. In particular, it has been observed that a sharp corner edge, such as on the edge of an anterior optic surface of an IOL optic, may exacerbate the undesired refractive effects. For example, an IOL optic edge profile, peripheral components besides optics, and in some cases the IOL optic surface can introduce the unwanted light. Such visual effects may be detrimental to IOL implantees, such as when the IOL optic edge profile was not designed to mitigate or even eliminate positive dysphotopsia.

As will be described in further detail, a hybrid edge comprising a concave curved portion and a straight portion is used to reduce or eliminate undesired refractive effects in an IOL optic. The hybrid IOL optic with the hybrid edge may eliminate glare or other undesired effects that adversely impact quality of vision.

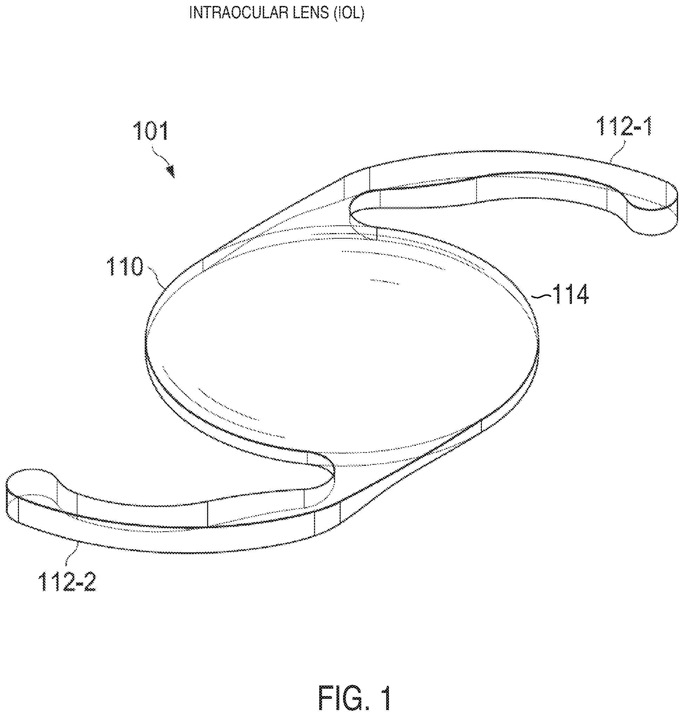

In FIGURE 1, IOL 101 may represent any kind of IOL used in ophthalmology. As shown, IOL 101 includes an optic zone 110 (also referred to herein as an 'IOL optic', or simply an 'optic') and two haptics 112-1, 112-2, which are shown in an exemplary configuration for descriptive purposes. It is noted that IOL 101 is a schematic illustration and is not drawn to scale or perspective. In various implementations, IOL 101 may include different types and numbers of haptics 112. In some implementations, IOL 101 may have no haptics. The materials used for optic zone 110 and haptics 112 may vary. For example, IOL 101 may be a non-foldable rigid IOL, such as with optic zone 110 comprising a polymethyl methacrylate (PMMA) lens. In some implementations, IOL 101 may be a flexible IOL, in which optic zone 110 may be comprised of various materials, such as silicone, hydrophobic acrylic, hydrophilic acrylic, hydrogel, collamer or combinations thereof. In IOL 101, haptics 112 may also be comprised of various materials, such as polypropylene, PMMA, hydrophobic acrylic, hydrophilic acrylic, silicone or combinations thereof.

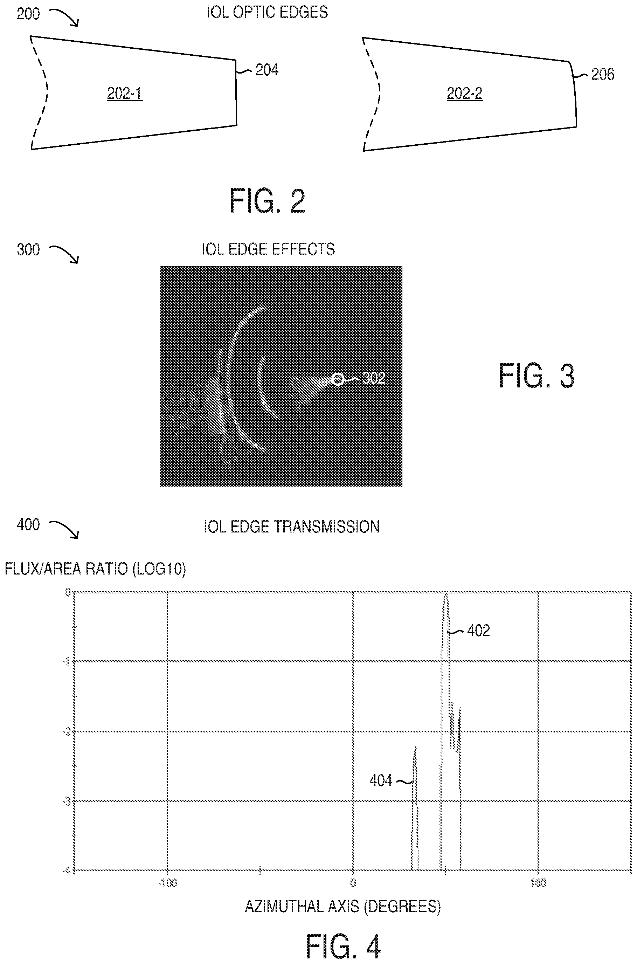

When an edge portion 114 of IOL 101 includes only sharp edges, such as edges with a very small or zero radius, or edges having a discontinuous slope with respect to the two surfaces forming the edge, it has been observed that IOL 101 may generate undesired refractive errors that may adversely affect the quality of vision of an implantee using IOL 101. In particular, the undesired refractive errors from certain sharp edges may result in undesired visual effects, such as glare or starbursts or halos, that may be exacerbated at certain incident angles of light, and may be caused by refraction of a portion of the light onto the retina at different locations than light that normally forms the visual image (see also FIGURES 2-4). Therefore, an edge portion 114 of optic zone 110 may be implemented with a hybrid edge portion comprising a convex portion having a curvature adjacent to a flat portion, as described in further detail herein. When edge portion 114 is implemented with the hybrid edge portion disclosed herein, the undesired refractive errors may be substantially reduced or eliminated, which is desirable due to the resultant lack of degradation of the quality of vision of the implantee.

Referring now to FIGURE 2, a depiction 200 of two examples of IOL optic edges 202 are shown. It is noted that IOL optic edges 202 are schematic illustrations and are not drawn to scale or perspective. In IOL optic 202-1, edge portion 204 is formed with a straight edge that results in two sharp corners having a very small or zero radius. The corners at the straight edge of edge portion 204 have a discontinuous slope with respect to the anterior and posterior faces of IOL optic 202-1 and may generate undesired refractive errors, such as with incoming light at certain angles of incidence (see also FIGURE 4).

In IOL optic 202-2, edge portion 206 is formed with a single curved edge that also results in two sharp corners having a very small or zero radius. The corners at the single curved edge of edge portion 206 have a discontinuous slope with respect to the anterior and posterior faces of IOL optic 202-2 and may also generate undesired refractive errors, such as with incoming light at certain angles of incidence.

Referring now to FIGURE 3, an image 300 showing IOL edge effects for IOL optic edges 200 (see FIGURE 2) is shown. In FIGURE 3, image 300 is generated using a comprehensive non-sequential ray tracing simulation in a model eye. In image 300, light that forms the visual image viewable using an IOL optic is shown by circle 302 as an annotation to image 300. The remaining portions of light outside of circle 302 in image 300 may represent refractive errors resulting from IOL edge effects. These remaining portions of light outside of circle 302 in image 300 may represent undesired visual effects that adversely affect the quality of vision of the implantee, as noted above.

Referring now to FIGURE 4, a plot 400 of IOL edge transmission is shown. Plot 400 is generated using an experimental configuration on an optics bench to evaluate transmission properties and empirical performance of an IOL. These properties and performance may be evaluated using the model eye and simulation discussed above in reference to FIGURE 3. The experiment may include simulating light passing through a model eye onto a model retina. The light may be simulated with various azimuthal angles to evaluate performance of an IOL for various conditions. When the incident light is simulated with a azimuthal angle equal to zero, a perfect image may be achieved on the retina in the model eye. As the azimuthal angle is varied throughout the experiment, adverse effects on the image and thus the performance of the IOL may be visualized. Specifically, plot 400 shows a flux/area ratio in a log10 scale versus an azimuthal angle of incident light in degrees for light passing through an IOL optic having only sharp edges, such as IOL optics 202 (see FIGURE 2). In plot 400, incident light is projected at about 50 degrees azimuthally and the light corresponding to the visual image is shown by peak 402, which represents the desired output from the IOL optic. However, due to the refractive errors of the sharp edges in the IOL optic, peak 404 shows undesired refraction that may result in a glare under certain conditions, which is undesirable and represents an adverse impact on the quality of vision using the IOL optic.

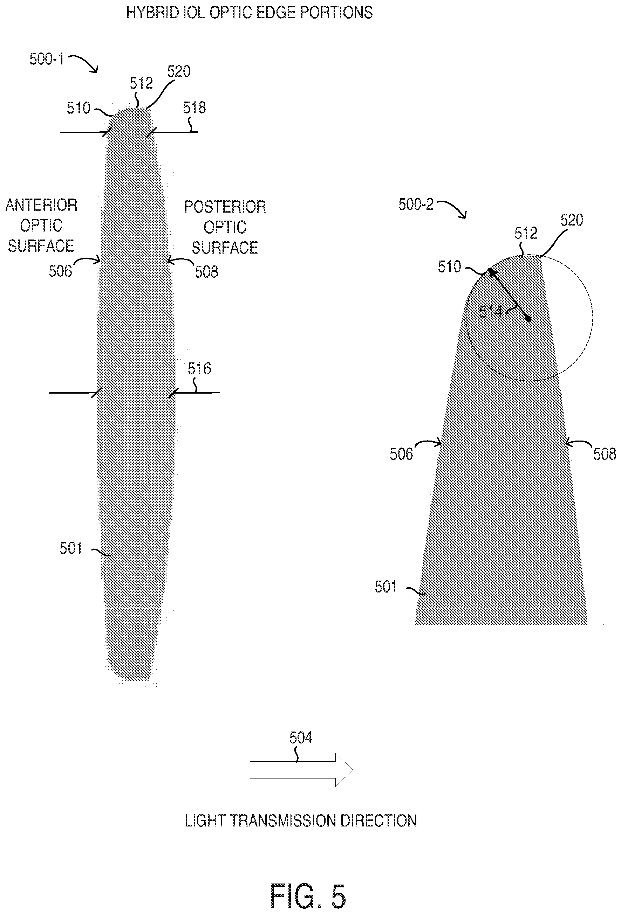

Referring now to FIGURE 5, depictions 500 of a hybrid edge of an IOL optic 501 are shown. A cross-sectional view of IOL optic 501 is shown by 500-1, while a magnified view with additional details of a convex portion 510 is shown by 500-2. It is noted that depictions 500 are schematic illustrations and are not drawn to scale or perspective.

500-1 shows a cross-section along a diameter of a circular hybrid IOL optic 501. IOL optic 501 is depicted as a concave lens having an anterior optic surface 506 and a posterior optic surface 508, such that light passes through IOL optic 501 in a light transmission direction 504, shown by an arrow in FIGURE 5, when implanted and in use. As shown, anterior optic surface 506 has a larger diameter concave shape than posterior optic surface 508. It will be understood that in various implementations, a surface contour of either anterior optic surface 506 or posterior optic surface 508 may vary and may comprise a convex surface, a concave surface, or a combination thereof. In some implementations, either anterior optic surface 506 or posterior optic surface 508 or both may have an irregular surface or a customized patterned surface for particular non-linear refractive performance. As shown in 500-1, a center thickness 516 of IOL optic 501 is a greater thickness than an edge thickness 518. The hybrid edge extends between the anterior optic surface 506 and the posterior optic surface 508 and includes a convex portion 510 and a flat surface portion 512, the flat surface portion 512 intersecting the posterior optic surface 508 so as to form a corner edge 520.

Anterior optic surface 506 may terminate at convex portion 510 (i.e., convex portion 510 may extend from a periphery of anterior optic surface 506). Convex portion 510 may have a curvature of a radius 514, as indicated by a circle having radius 514 that is superimposed over convex portion 510. It is noted that various values for radius 514 may be used. In particular embodiments, it has been observed that values for radius 514 between 0.4 mm and 0.6 mm may be particularly desirable for the geometry and size ranges of IOL optic 501 that are implanted in different individuals, as also determined relative to a diameter of IOL optic 501 and diameters of certain eye structures, such as a natural lens or a capsular bag that receives IOL optic 501. Specifically, the mitigation of glare, positive dysphotopsia and unwanted light from the IOL optic may depend on the edge profile and an arc geometry of the IOL optic. A smaller value for radius 514, less than about 0.4 mm, may not sufficiently mitigate the glare component of the undesired refraction, while a larger value for radius 514, greater than about 0.6 mm, may not be feasible with common ranges of edge thickness 518 and may be constrained by a maximum value of edge thickness 518.

Posterior optic surface 508 may terminate at flat portion 512 (i.e., flat portion 512 may extend from a periphery of posterior optic surface 508) so as to form corner 520. Moreover, flat portion 512 may intersect convex portion 510 so as to form the hybrid optic edge. Corner edge 520 may be implemented as a sharp edge, such as having a very small or zero diameter, or which is discontinuous in slope with respect to flat surface portion 512 and posterior optic surface 508, which meet at corner edge 520.

In FIGURE 5, due to the particular shape of edge portions 500, refractive errors may be avoided with IOL optic 501, such that negligible or no undesired refraction occurs that may result in a glare under certain conditions, which is desirable for maintaining the quality of vision using IOL optic 501.

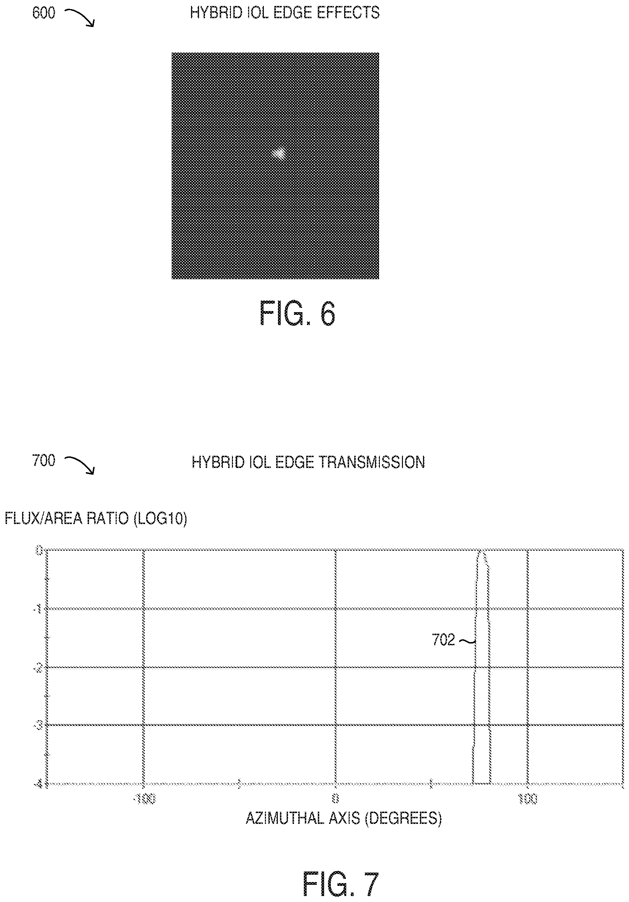

Referring now to FIGURE 6, an image 600 showing edge effects for the hybrid optic edge design described with respect to FIGURE 5 is shown. In image 600, light that forms the visual image viewable using hybrid IOL optic 501 appears as a single central field, while no other light is visible, indicating no undesired visual effects that adversely affect the quality of vision of the implantee, as noted above (compare also with FIGURE 3).

Referring now to FIGURE 7, a plot 700 of IOL edge transmission for the hybrid optic edge design described with respect to FIGURE 5 is shown. Specifically, plot 700 shows a flux/area ratio in a log10 scale versus an azimuthal angle of incident light in degrees for light passing through hybrid IOL optic 501 (see FIGURE 5). In plot 700, incident light is projected at about 75 degrees azimuthally and the light corresponding to the visual image is shown by peak 702, which represents the desired output from the IOL optic. NO other peaks are present in plot 700, indicating that no undesired refraction occurs using hybrid IOL 501 that may result in a glare under certain conditions.

As disclosed herein, a hybrid edge comprising a concave curved portion and a straight portion is used to reduce or eliminate undesired refractive effects, such as positive dysphotopsia, in an IOL optic. The hybrid IOL optic with the hybrid edge may eliminate glare or other undesired effects that adversely impact quality of vision.

## Claims

1. An intraocular lens comprising:
an optic (501) comprising:
an anterior optic surface (506),
a posterior optic surface (508), and
an optic edge extending between the anterior optic surface and a posterior optic surface; wherein the optic edge is a hybrid optic
edge comprising:
a first convex surface portion (510) extending from a periphery of the anterior optic surface (506), and having a first radius of curvature (514) between 0.4 mm and 0.6 mm.; and
a second flat surface portion (512) extending from a periphery of the posterior optic surface (508) so as to form a corner edge (520), said second flat surface portion (512) being configured to intersect the convex surface portion (510) so as to form the hybrid optic edge.

2. The intraocular lens of claim 1, wherein the first radius of curvature (514) of the convex portion is greater than a second radius of the corner edge (520).

3. The intraocular lens of claim 2, wherein the first radius of curvature (514) is less than a third radius of curvature of the anterior optic surface (506) at a central portion of the intraocular lens.

4. The intraocular lens of claim 3, wherein the first radius of curvature (514) is less than a fourth radius of curvature of the posterior optic surface (508) at a central portion of the intraocular lens, and wherein the third radius of curvature is greater than the fourth radius of curvature.

5. The intraocular lens of claim 2, wherein the second radius of curvature of the corner edge (520) is zero.

6. The intraocular lens of claim 1, wherein the corner edge (520) has a discontinuous slope with respect to the slopes respectively of the flat surface portion (512) and the posterior optic surface (508).

7. The intraocular lens of claim 1, wherein the intraocular lens has a center thickness (516) that is greater than an edge thickness (518) of the optic edge.

## Patentansprüche

1. Intraokularlinse, umfassend:
eine Optik (501), umfassend:
eine anteriore optische Fläche (506),
eine posteriore optische Fläche (508) und
einen optischen Rand, der sich zwischen der anterioren optischen Fläche und einer posterioren optischen Fläche erstreckt; wobei der optische Rand ein hybrider optischer Rand ist, der Folgendes umfasst:
einen ersten, konvexen Flächenanteil (510), der sich von einer Umfangslinie der anterioren optischen Fläche (506) erstreckt und einen ersten Krümmungsradius (514) zwischen 0,4 mm und 0,6 mm aufweist; und
einen zweiten, ebenen Anteil (512), der sich von einer Umfangslinie der posterioren optischen Fläche (508) erstreckt, um somit einen Kurvenrand (520) zu bilden, wobei der zweite, ebene Flächenanteil (512) dafür ausgelegt ist, sich mit dem konvexen Flächenanteil (510) zu schneiden, um somit den hybriden optischen Rand zu bilden.

2. Intraokularlinse nach Anspruch 1, wobei der erste Krümmungsradius (514) des konvexen Anteils größer als ein zweiter Radius des Kurvenrands (520) ist.

3. Intraokularlinse nach Anspruch 2, wobei der erste Krümmungsradius (514) kleiner als ein dritter Krümmungsradius der anterioren optischen Fläche (506) an einem Zentralanteil der Intraokularlinse ist.

4. Intraokularlinse nach Anspruch 3, wobei der erste Krümmungsradius (514) kleiner als ein vierter Krümmungsradius der posterioren optischen Fläche (508) an einem Zentralanteil der Intraokularlinse ist, und wobei der dritte Krümmungsradius größer als der vierte Krümmungsradius ist.

5. Intraokularlinse nach Anspruch 2, wobei der zweite Krümmungsradius des Kurvenrands (520) null ist.

6. Intraokularlinse nach Anspruch 1, wobei der Kurvenrand (520) bezüglich den jeweiligen Steigungen des ebenen Flächenanteils (512) und der posterioren optischen Fläche (508) eine unstetige Steigung aufweist.

7. Intraokularlinse nach Anspruch 1, wobei die Intraokularlinse eine Zentrumsdicke (516) aufweist, die größer als eine Randdicke (518) des optischen Rands ist.

## Revendications

1. Lentille intraoculaire comprenant :
une optique (501) comprenant :
une surface optique antérieure (506),
une surface optique postérieure (508), et
un bord optique s'étendant entre la surface optique antérieure et une surface optique postérieure ; dans laquelle le bord optique est un bord optique hybride comprenant :
une première partie de surface convexe (510) s'étendant depuis une périphérie de la surface optique antérieure (506), et ayant un premier rayon de courbure (514) compris entre 0,4 mm et 0,6 mm ; et
une deuxième partie de surface plate (512) s'étendant depuis une périphérie de la surface optique postérieure (508) de manière à former un bord d'angle (520), ladite deuxième partie de surface plate (512) étant configurée pour croiser la partie de surface convexe (510) de manière à former le bord optique hybride.

2. Lentille intraoculaire de la revendication 1, dans laquelle le premier rayon de courbure (514) de la partie convexe est supérieur à un deuxième rayon de courbure du bord d'angle (520).

3. Lentille intraoculaire de la revendication 2, dans laquelle le premier rayon de courbure (514) est inférieur à un troisième rayon de courbure de la surface optique antérieure (506) au niveau d'une partie centrale de la lentille intraoculaire.

4. Lentille intraoculaire de la revendication 3, dans laquelle le premier rayon de courbure (514) est inférieur à un quatrième rayon de courbure de la surface optique postérieure (508) au niveau d'une partie centrale de la lentille intraoculaire, et dans laquelle le troisième rayon de courbure est supérieur au quatrième rayon de courbure.

5. Lentille intraoculaire de la revendication 2, dans laquelle le deuxième rayon de courbure du bord d'angle (520) est nul.

6. Lentille intraoculaire de la revendication 1, dans laquelle le bord d'angle (520) a une pente discontinue par rapport aux pentes respectivement de la partie de surface plate (512) et de la surface optique postérieure (508) .

7. Lentille intraoculaire de la revendication 1, la lentille intraoculaire ayant une épaisseur au centre (516) qui est supérieure à une épaisseur de bord (518) du bord optique.
